# EUROPÄISCHE PATENTSCHRIFT

(11) **EP 1 595 884 B1**
(45) Veröffentlichungstag und Bekanntmachung des Hinweises auf die Patenterteilung: **08.06.2011**
(21) Anmeldenummer: 05009790.6
(22) Anmeldetag: 13.02.2004
(51) Int. Cl.: C07D 495/04, A61K 31/435, A61K 9/14

(54) **Salz einer Sulfonsäure mit Clopidogrel und dessen Verwendung zur Herstellung pharmazeutischer Formulierungen**
SALT OF A SULFONIC ACID CONTAINING CLOPIDOGREL AND USE THEREOF FOR THE PRODUCTION OF PHARMACEUTICAL FORMULATIONS
SEL D'UN ACIDE SULFONIQUE COMPRENANT DU CLOPIDOGREL ET SON UTILISATION POUR PRODUIRE DES FORMULATIONS PHARMACEUTIQUES

(30) Priorität: 13.02.2003 DE 10305984
(43) Veröffentlichungstag der Anmeldung: 16.11.2005
(62) Teilanmeldung aus: 04710847.7
(73) Patentinhaber: Helm AG, 20097 Hamburg (DE)
(72) Erfinder: Doser, Karlheinz, Dr., 21244 Buchholz i.d. Nordheide (DE); Glänzer, Klaus, Dr., 22337 Hamburg (DE)
(74) Vertreter: Becker, Eberhard

(56) Entgegenhaltungen:
- EP-A- 1 161 956
- WO-A-2004/013147
- WO-A-2004/106344
- US-A- 4 847 265
- US-B1- 6 284 277
- US-B1- 6 509 348

## Beschreibung

Die vorliegende Erfindung Clopidogrel-Besylat x ½ Dioxan, ein Verfahren zu dessen Herstellung sowie dessen Verwendung zur Herstellung pharmazeutischer Formulierungen.

Clopidogrel (5-Methyl-α-(4,5,6,7-tetrahydro[2,3-c]thienopyridyl)(2-chlorphenyl)acetat) ist als Wirkstoff aus EP-A-0 099 802 bekannt. Clopidogrel wirkt als Plättchenaggregationshemmer und kann daher beispielsweise zur Prävention thromboembolischer Ereignisse, wie z.B. Schlaganfall oder Myokardinfarkt, eingesetzt werden.

Die EP-A-0 281 459 schlägt vor, in pharmazeutischen Formulierungen anorganische Salze des (S)-(+)-Clopidogrels einzusetzen, insbesondere (S)-(+)-Clopidogrel-Hydrogensulfat. Dieses Dokument offenbart auch organische Salze des Clopidogrels, diese werden jedoch als amorph und/oder hygroskopisch sowie schwer zu reinigen beschrieben.

Das in pharmazeutischen Formulierungen eingesetzte (S)-(+)-Clopidogrel-Hydrogensulfat weist den Nachteil auf, dass zu seiner Herstellung konzentrierte Schwefelsäure benötigt wird und dass entsprechende Produkte wegen des aziden Protons stark sauer reagieren. Diese sauren Eigenschaften beeinflussen die Kompatibilität mit vielen pharmazeutischen Hilfsstoffen und damit die Stabilität entsprechender Arzneiformen negativ. Es besteht daher ein Bedürfnis nach stabilen, leicht zu reinigenden und leicht mit verschiedenen pharmazeutischen Hilfsstoffen, wie Arzneimittelträgem und -zusatzstoffen, verarbeitbaren Formen von Clopidogrel.

Eine Aufgabe der vorliegenden Erfindung besteht somit darin, Clopidogrel in einer Form zur Verfügung zu stellen, die leicht zu reinigen, stabil und auch in industriellem Maßstab leicht zu verarbeiten ist. Außerdem sollen Wechselwirkungen mit üblichen Arzneimittelträgern, -zusatzstoffen und -verarbeitungshilfsstoffen möglichst vermieden werden.

Es wurde nun überraschend gefunden, dass sich entgegen der Offenbarung der EP-A-0 281 459 das Salz der Benzolsulfonsäure mit Clopidogrel unter bestimmten Bedingungen zur Herstellung von pharmazeutischen Formulierungen eignet.

Die vorliegende Erfindung betrifft somit das Salz der Benzolsulfonsäure mit Clopidogrel, das zumindest teilweise in kristalliner Form vorliegt. Die vorliegende Erfindung betrifft außerdem das Salz der Benzolsulfonsäure mit Clopidogrel, herstellbar durch Fällen des Salzes aus einer Clopidogrel-Lösung, wobei das Lösungsmittel Dioxan umfasst.

Als Clopidogrel kann erfindungsgemäß ein racemisches Gemisch der beiden Clopidogrel-Isomere eingesetzt werden. Alternativ können die reinen Isomere verwendet werden, wobei das (S)-(+)-Clopidogrel-Isomer bevorzugt ist.

Erfindungsgemäß wurde überraschend gefunden, dass es entgegen der Lehre der EP-A-0 281 459 möglich ist, das Salz der Benzolsulfonsäure mit Clopidogrel in pharmazeutischen Formulierungen und insbesondere in oral zu verabreichenden pharmazeutischen Formulierungen einzuarbeiten. Die Erfindung umfasst somit auch die Verwendung des Salzes der Benzolsulfonsäure mit Clopidogrel zur Herstellung einer pharmazeutischen Formulierung sowie pharmazeutische Formulierungen, die ein solches Salz enthalten.

Das erfindungsgemäße Salz ist vollständig kristallin. In dieser Form lässt sich das Salz leichter reinigen als in den in der EP-A-0 281 459 offenbarten amorphen Formen. Außerdem lässt sich kristallines Salz leichter zu pharmazeutischen Formulierungen weiterverarbeiten.

Erfindungsgemäß wurde ferner gefunden, dass sich die gewünschten kristallinen Salze einer Sulfonsäure mit Clopidogrel einfach und in einer für die Weiterverarbeitung zu einer pharmazeutischen Formulierung günstigen Form durch Fällen des Salzes aus einer Lösung von Clopidogrel herstellen lassen, wenn das Lösungsmittel Dioxan umfasst.

In einer Ausführungsform können sowohl die Clopidogrel-Base als auch die Benzolsulfonsäure in Dioxan gelöst, vermischt und das gewünschte Salz durch Zugabe von Ethylacetat ausgefällt werden.

Nach dem vorstehend beschriebenen Verfahren lässt sich das Salz der Benzolsulfonsäure mit Clopidogrel in guter Ausbeute und Reinheit erhalten, so dass sich dieses Salz, insbesondere wenn es in kristalliner Form vorliegt, besonders zur Herstellung von pharmazeutischen Formulierungen eignet.

Es wurde außerdem gefunden, dass das Salz der Benzolsulfonsäure mit Clopidogrel dann besonders vorteilhafte der Benzols Eigenschaften beispielsweise in Bezug auf seine Kristallinität aufweist, wenn es Lösungsmittelmoleküle enthält. Die in das Salz als Solvat eingelagerten Lösungsmittelmoleküle stammen aus der Lösung, aus der das Salz ausgefällt worden ist.

Das aus Dioxan ausgefällte Benzolsulfonsäure-Salz enthält Dioxanmoleküle. Die 10 intensitätsstärksten Peaks des Röntgenpulverspektrums dieses Salzes weisen die folgenden 2Θ -Werte auf:

| relative Intensität | 2Θ |
|---|---|
| 51,66 | 10,78 |
| 54,15 | 10,87 |
| 90,13 | 12,13 |
| 50,83 | 14,34 |
| 50,27 | 16,43 |
| 76,03 | 21,57 |
| 81,19 | 22,87 |
| 100,00 | 23,06 |
| 54,18 | 23,72 |
| 54,05 | 25,17 |

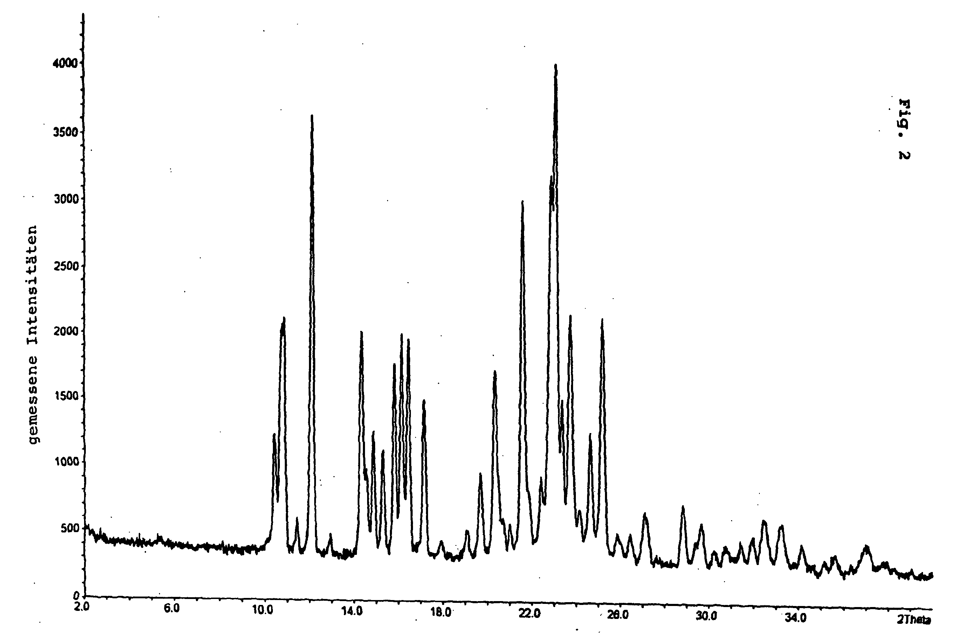

Das wie vorstehend beschrieben gemessene Röntgenpulverspektrum dieses Salzes ist in anliegender Figur 2 wiedergegeben.

Darüber hinaus wurde gefunden, dass das Salz der Benzolsulfonsäure mit Clopidogrel im Vergleich mit anderen Clopidogrel-Salzen in besonders hoher Reinheit gewonnen wird. Beim Auskristallisieren aus Dioxan wird beispielsweise ein Besylat-Salz mit nur 0,085% Verunreinigungen (nach HPLC) erhalten. Daher eignet sich das erfindungsgemäße Salz für die Herstellung von reinem Clopidogrel. Die vorliegende Erfindung betrifft daher auch ein Verfahren zur Reinigung von Clopidogrel, wobei verunreinigtes Clopidogrel oder ein Salz davon, gegebenenfalls nach Freisetzung der Clopidogrel-Base, in das Salz einer Sulfonsäure mit Clopidogrel überführt wird und, falls gewünscht, anschließend aus dem isolierten Salz der Sulfonsäure die Clopidogrel-Base freigesetzt und/oder in ein anderes Salz überführt wird. Erfindungsgemäss wird das Besylat-Salz verwendet.

Ein weiterer Aspekt der vorliegenden Erfindung besteht darin, Clopidogre-Besylat x ½ Dioxan in einer einfach weiterverarbeitbaren Form zur Verfügung zu stellen. Dies wird erfindungsgemäß dadurch erreicht, dass das Salz auf ein festes Adsorbtionsmittel aufgebracht wird. Hierdurch werden Wirkstoffpartikel erhalten, die sich leicht schütten und dosieren lassen.

Als Adsorbtionsmittel eignet sich jeder physiologisch und pharmazeutisch akzeptable, vorzugsweise partikuläre Feststoff, der in der Lage ist, Clopidogre-Besylat zu adsorbieren. Vorzugsweise ist der Feststoff ein rieselfähiges Pulver, das leicht zu oralen pharmazeutischen Formulierungen weiterverarbeitet werden kann.

Physiologisch und pharmazeutisch akzeptable Adsorbtionsmittel sind beispielsweise:
1. Natürliche oder aufbereitete Adsorbtionsmittel aus der Gruppe der Tonerden (Tonmaterialien) und sonstiger Erden und Mineralien, z.B. Attapulgite, Aluminium-Magnesium-Silikate (Carrisorb®, Gelsorb®), Magnesium-Aluminium-Silikate (Pharmasorb®, Veegum®), Magnesiumsilikate (Talkum), Calciumsilikate, Bentonite, Kaolin, Magnesium-Trisilikate, Montmorillonite, Porzellanerden (Bolus), Sepiolite (Meerschaum)
2. Kieselgele, Kieselgur, Kieselsäuren
3. Kolloidale (hochdisperse) Kieselsäuren (hydrophobe oder hydrophile Aerosile®, Cab-o-sile®)
4. Cellulosen, modifizierte Cellulosen, fein- und mikrokristalline Cellulosen sowie Cellulosederivate, Celluloseacetat, Cellulosefettsäureester, Cellulosenitrate, Celluloseether (Carboxymethylcellulosen, Ethylcellulosen, Hydroxyethylcellulosen, Hydroxypropylcellulosen, Methylcellulosen, Methylethylcellulosen, Methylhydroxypropylcellulosen)
5. Zucker und Zuckerderivate (Mono- und Polysaccharide), Lactosen, Dextrane, Dextrose, Cyclodextrine
6. Native Mais-, Reis-, Tapioka-, Weizen-, Kartoffelstärken und deren Derivate, Dextrine, prägelatinisierte, ganz oder teilweise hydrolysierte Stärken
7. Feste Polyole, insbesondere Mannit oder Sorbit
8. Polyacrylate, Acrylsäurepolymerisate bzw. Copolymerisate
9. Phosphate, Sulfate, Carbonate, Gluconate, Oxide von Alkali- und Erdalkalimetallen sowie physiologisch akzeptablen Schwer- und Übergangsmetallen
10. Guar-Mehl, Guar-Gummi
11. Johannisbrotmehl (Carob-Mehl, Carob-Gummi)
12. Alginsäure, Alginate und Algenmehl
13. Tragant
14. Carbo vegetabilis (Kohle)
15. Pektine und Amylopektine
16. N-Vinylpyrrolidon-Polymere, wie z.B. Povidon oder Crospovidon.

Die Adsorbtionsmittel können einzeln oder in Mischung aus zwei oder mehr Adsorbtionsmitteln eingesetzt werden. Außerdem können die erfindungsgemäßen Wirkstoffpartikel neben dem Adsorbtionsmittel übliche pharmazeutische Hilfsstoffe beispielsweise zur Herstellung von Direkttablettiermischungen bzw. zur Herstellung von Granulaten zur Weiterverarbeitung zu Arzneimitteln umfassen. Alternativ können die erfindungsgemäßen Wirkstoffpartikel nach ihrer Herstellung mit entsprechenden Hilfsstoffen vermischt und dann zu pharmazeutischen Formulierungen weiterverarbeitet werden.

Besonders bevorzugte Adsorbtionsmittel sind bestimmte Lactosen (z. B. Lactopress®), bestimmte Mannite (z. B. Mannogem®) und bestimmte Cellulosen (z. B. Celphere®), insbesondere Lactopress®. Ein Granulat auf Basis von pyrogen hergestelltem Siliciumdioxid wird, obwohl möglich, vorzugsweise nicht als Trägermedium eingesetzt.

Zur Desorptionssteuerung können geeignete Netzmittel eingesetzt werden. Zur Stabilitätsverbesserung können beispielsweise Antioxidantien, wie z.B. Ascorbinsäure und deren Salze, zugefügt werden. Weitere geeignete Hilfsmittel sind Emulgatoren, Lösungsmittel und Lösungsvermittler.

Die erfindungsgemäßen Wirkstoffpartikel können beispielsweise aus einem Lösungsmittel gewonnen werden, in dem das Adsorbtionsmittel unlöslich oder schwer löslich und das Clopidogrel-Besylat löslich ist. Hierzu kann das Adsorbtionsmittel in dem Lösungsmittel suspendiert werden. Vor ode nach dem Suspendierungsschritt kann das Clopidogrel-Besylat in dem Lösungsmittel gelöst werden. Der Wirkstoff kann dabei entweder direkt-Besylat oder als Lösung in demselben oder einem anderen Lösungsmittel zugegeben werden. Anschließend werden die Wirkstoffpartikel, die das Clopidogrel-Besylat auf dem Adsorbtionsmittel aufgebracht umfassen, aus dem Lösungsmittel beispielsweise durch Verdampfen des Lösungsmittels gewonnen.

Als Lösungsmittel eignen sich alle üblichen Lösungsmittel, in denen das gewählte Adsorbtionsmittel nicht löslich oder schwer löslich und das Clopidogrel-Besylat löslich ist. Beispielsweise können die vorstehend-Besylat zur Herstellung des Salzes beschriebenen Lösungsmittel verwendet werden.

In einer alternativen Ausführungsform des erfindungsgemäßen Verfahrens zur Herstellung von Wirkstoffpartikeln führt man die letzte Stufe der Synthese von Clopidogrel in Gegenwart des Adsorbtionsmittels durch. Dadurch können die gewünschten Wirkstoffpartikel ohne einen isolierenden Zwischenschritt hergestellt werden. Ferner können beispielsweise Clopidogrel und Benzolsulfonsäure mit der Suspension des Adsorbtionsmittels vermischt werden. Dabei können das Clopidogrel und die Säure jeweils getrennt in einem Lösungsmittel aufgelöst und gleichzeitig oder nacheinander zu der Suspension zugegeben werden. Alternativ können das Clopidogrel und Benzolsulfonsäure in reiner Form zu der Suspension zugegeben werden. Einzelne Bestandteile können auch separat vorgemischt und dann gemeinsam zu der Suspension zugegeben werden.

Das Gewichtsverhältnis von Adsorbtionsmittel zu darauf adsorbiertem Clopidogrel oder einem Salz davon ist für die vorliegende Erfindung nicht besonders wesentlich und kann vom Fachmann in Abhängigkeit von dem gewünschten Verwendungszweck frei gewählt werden. Bei einer Weiterverarbeitung zu oralen, pharmazeutischen Formulierungen sollte jedoch beachtet werden, dass genügend Clopidogrel auf dem Adsorbtionsmittel aufgebracht ist, damit die gewünschte Dosierung in der Einheitsdosisform erreicht wird. Beispielsweise kann das Gewichtsverhältnis von Clopidogrel-Besylat des Clopidogrels, bezogen auf die freie Clopidogrel-Base, zu Adsorbtionsmittel im Bereich von 2:1 bis 1:6 liegen (d.h. z. B. 1 Gewichtsteil Clopidogrel-Base pro 6 Gewichtsteile Adsorbtionsmittel), vorzugsweise im Bereich von 1:1 bis 1:3.

Die vorliegende Erfindung wird durch die nachfolgenden Beispiele näher erläutert, ohne sie auf diese einzuschränken.

In den Beispielen wurden die Röntgenpulverspektren mit einem STOE STADI P Transmissionsdiffraktometer mit Kupfer-Kα-Strahlung, die NMR-Daten mit einem Varian Unityplus 300-Gerät und die CHN-Daten mit einem Carlo Erba Analyzer 1106 aufgenommen.

### Beispiel 1 (nicht erfindungsgemäß)

### Darstellung von Clopidogrel-Benzolsulfonat aus Aceton/Toluol

4,0 g (12,5 mmol) Clopidogrel-Base wurden in 30 ml Toluol gelöst und dazu 2,0 g (12,5 mmol) wasserfreie Benzolsulfonsäure in 10 ml Aceton gegeben. Nach einiger Zeit und Verreiben mit einem Glasstab wird das Produkt fest und läßt sich absaugen. Das Produkt wurde über Nacht getrocknet unter Vakuum am Pumpenstand im Exsiccator.
Ausbeute: 67 % Fp. 87° - 90° C
NMR (ppm)
2.35 (Toluol), 3.0 - 3.5 und 3.8 - 4.3 (4 H), 3.79 (3 H), 4.8 - 5.2 (1 H), 5.69(1 H), 6.6 - 6.8 (1 H), 7.2 - 8.0 (12H)

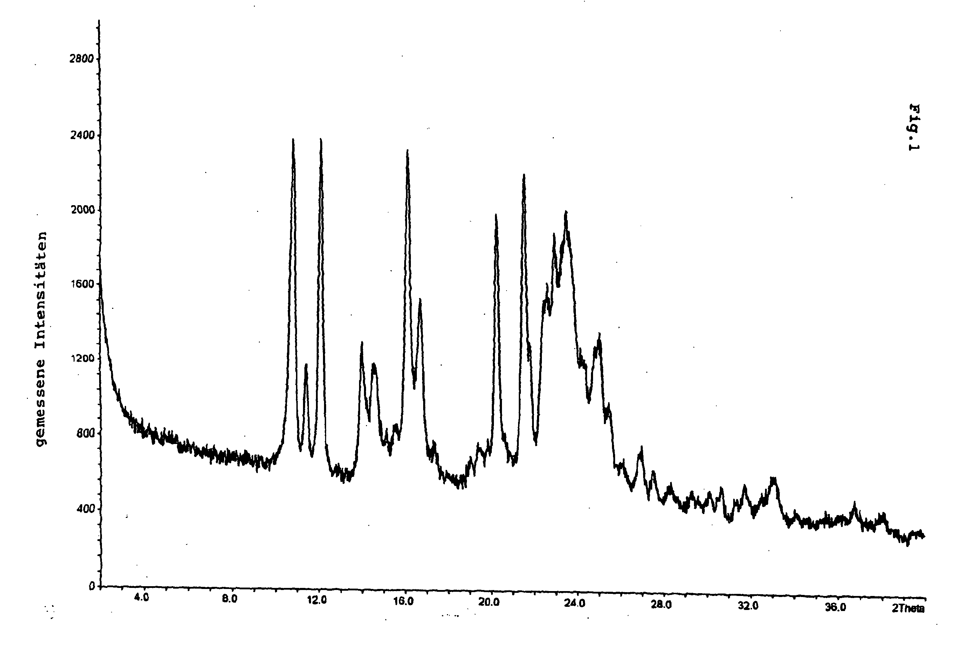

Das Röntgenpulverspektrum dieses Salzes ist in Figur 1 wiedergegeben.

Beim weiteren Trocknen bis zur vollständigen Entfernung des Toluols aus dem Salz bricht die Kristallstruktur zusammen und man erhält amorphes Clopidogrel-Benzolsulfonat.

### Beispiel 2

### Darstellung von Clopidogrel-Benzolsulfonat aus Dioxan

109,2 g (339,7 mmol) Clopidogrel-Base, in 300 ml Dioxan gelöst, werden bei 10° C unter Rühren mit einer Lösung von 53,7 g (339,7 mmol) wasserfreier Benzolsulfonsäure in 100 ml Dioxan versetzt. Zu dieser Lösung gibt man 250 ml Ethylacetat und stellt diese Lösung in den Tiefkühlschrank über Nacht. Man lässt die Lösung auf Raumtemperatur erwärmen, saugt das Kristallisat ab und wäscht mit Ethylacetat nach. Das Produkt wird unter Vakuum bei Raumtemperatur 48 Std. getrocknet.
Ausbeute: 71 % Fp. 93° - 95° C

| Elementaranalyse | | | |
|---|---|---|---|
| Werte [%] | berechnet für Clopidogrel-Besylat *1/2 Dioxan | gefunden | |
| C | 55,01 | 55,28 | 55,03 |
| H | 5,00 | 5,12 | 4,99 |
| N | 2,67 | 2,62 | 2,53 |

NMR (ppm)
3.0 - 3.5 und 3.8 - 4.3 (4 H), 3.79 (3 H), 4.8 - 5.2 (1 H), 5,68 - 5,72 (1 H), 6.6 - 6.8 (1 H), 7.2 - 8.0 (12H), 3,70 (4 H; ½ Dioxan)

Das Röntgenpulverspektrum dieses Salzes ist in Figur 2 wiedergegeben.

### Beispiel 3 (nicht erfindungsgemäß)

### Darstellung von Clopidogrel-Toluolsulfonat aus MTB-Ether

4,0 g (12,5 mmol) Clopidogrel-Base löst man 50 ml Ethylacetat und setzt dazu eine Lösung von 2,2 g (12,5 mmol) Toluolsulfonsäure (wasserfrei) in 30 ml Ethylacetat. Man destilliert davon im Vakuum ca. 50 ml Ethylacetat ab und setzt 150 ml MTB-Ether und 5 ml Isopropanol zu und verrührt den Rückstand zu einer festen Masse. Man saugt ab und trocknet im Vakuum bei Raumtemperatur.
Ausbeute: 62 % Fp. 78° - 82° C

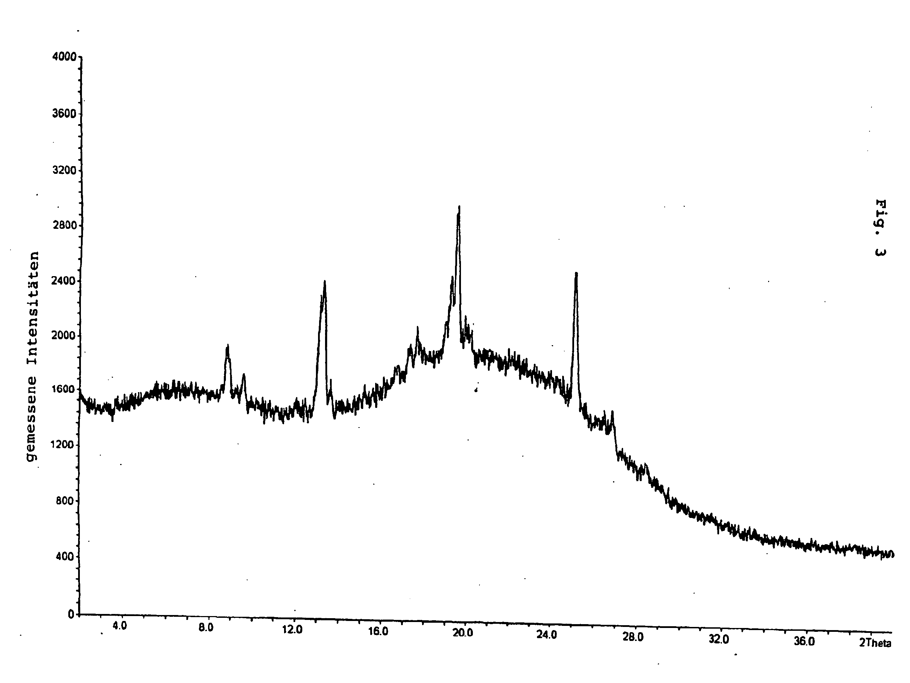

Das Röntgenpulverspektrum dieses Salzes ist in Figur 3 wiedergegeben.

### Beispiel 4

### Stabilitätsuntersuchungen

4.1 Die Stressstabilität verschiedener Salze des Clopidogrels wurde unter mehreren Bedingungen untersucht. Als Salze wurden die bislang als am stabilsten bekannte Form II des Clopidogrel-Hydrogensulfats, Clopidogrel-Hydrochlorid (hergestellt gemäß EP 281 459), amorphes Clopidogrel-Benzolsulfonat und kristallines Clopidogrel-Benzolsulfonat (aus vorstehendem Beispiel 2) eingesetzt. Folgende Versuche wurden durchgeführt:

### Stabilität unter sauren Bedingungen

50 mg des jeweiligen Salzes werden in einem volumetrischen Kolben (100 ml) eingewogen und mit 2 ml 1 N HCl versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur werden 2 ml 1 N NaOH zugegeben und es wird bis auf 100 ml mit der mobilen Phase aufgefüllt.

Das Ergebnis wird mittels HPLC bestimmt.

### Stabilität unter basischen Bedingungen

50 mg des jeweiligen Salzes werden in einem volumetrischen Kolben (100 ml) eingewogen und mit 2 ml 1 N NaOH versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur werden 2 ml 1 N HCl zugegeben und es wird bis auf 100 ml mit der mobilen Phase aufgefüllt.

Das Ergebnis wird mittels HPLC bestimmt.

### Stabilität unter oxidativen Bedingungen

50 mg des jeweiligen Salzes werden in einem volumetrischen Kolben (100 ml) eingewogen und mit 2 ml 3% H₂O₂ versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur wird bis auf 100 ml mit der mobilen Phase aufgefüllt.

Das Ergebnis wird mittels HPLC bestimmt.

### Stabilität unter neutralen Bedingungen

50 mg des jeweiligen Salzes werden in einem volumetrischen Kolben (100 ml) eingewogen und mit 2 ml Wasser versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur wird bis auf 100 ml mit der mobilen Phase aufgefüllt.

Das Ergebnis wird mittels HPLC bestimmt.

### Stabilität unter Wärmeeinfluss

50 mg des jeweiligen Salzes werden in einem volumetrischen Kolben (100 ml) eingewogen und 20 h bei 80° C aufbewahrt. Nach dem Ende des jeweiligen Experiments und Abkühlen auf Raumtemperatur wird bis auf 100 ml mit der mobilen Phase aufgefüllt.

Das Ergebnis wird mittels HPLC bestimmt.

Die HPLC-Messungen erfolgten in allen Fällen unter folgenden Bedingungen mit UV-Detektion:

| | | |
|---|---|---|
| Säule | Hypersil BDS 5 µm, 250 * 4.6 mm | |
| Mobile Phase | Methanol | 650 ml |
| | 0,05 M 1-Octansulfonsäure -Na-Salz | 350 ml |
| | (mit Triethylamin und Phosphorsäure auf pH 2,5 eingestellt). | |
| Flussrate | 1ml/min | |
| Säulentemperatur | Raumtemperatur | |
| Wellenlänge | 215 nm | |
| Injection volume | 20 µl | |
| Retentionszeit | ca 15 min | |

Die Ergebnisse dieser Untersuchungen sind in den folgenden Tabellen 1 - 4 zusammengefasst:

### Clopidogrel-Hydrogensulfat

**Tabelle 1:**

| **Bedingung** | **Raumtemperatur** | **80°C** |
|---|---|---|
| sauer | 0.32 % | 2.96 % |
| alkalisch | 0.32 % | 59.48 % |
| oxidierend | 0.33 % | 3.50 % |
| neutral | 0.40 % | 1.63 % |
| Wärme | --- | 0.31 % |

Clopidogrel-Hydrochlorid

**Tabelle 2:**

| **Bedingung** | **Raumtemperatur** | **80°C** |
|---|---|---|
| sauer | 1.86 % | 3.31 % |
| alkalisch | 1.86 % | 72.89 % |
| oxidierend | 1.83 % | 4.16 % |
| neutral | 1.84 % | 4.33 % |
| Wärme | --- | 32.43 % |

Clopidogrel-Benzolsulfonat (amorph)

**Tabelle 3:**

| **Bedingung** | **Raumtemperatur** | **80°C** |
|---|---|---|
| sauer | 0.64 % | 2.36 % |
| alkalisch | 0.64 % | 25.04 % |
| oxidierend | 0.83 % | 2.94 % |
| neutral | 0.85 % | 3.01 % |
| Wärme | --- | 11.52 % |

Clopidogrel-Benzolsulfonat (kristallin)

**Tabelle 4:**

| **Bedingung** | **Raumtemperatur** | **80°C** |
|---|---|---|
| sauer | 0.14 % | 2.76% |
| alkalisch | 0.14 % | 28.05% |
| oxidierend | 0.13 % | 3.98 % |
| neutral | 0.19 % | 4.18 % |
| Wärme | --- | 4.52 % |

Man erkennt, dass entgegen der Lehre des EP 281 459 das amorphe Clopidogrel-Benzolsulfonat im Vergleich zu den Hydrogensulfat- und Hydrochlorid-Salzen des Clopidogrels eine vergleichbare und insbesondere unter alkalischen Bedingungen wesentlich erhöhte Stabilität aufweist. Darüber hinaus ist die Stabilität der kristallinen Form des Clopidogrel-Benzolsulfonats gegenüber der amorphen Form dieses Salzes insbesondere bei der für die Lagerung von pharmazeutischen Produkten wichtigen Raumtemperatur weiter erhöht. Kristallines Clopidogrel-Benzolsulfonat ist sogar stabiler als das bislang als am stabilsten bekannte und in pharmazeutischen Formulierungen eingesetzte Clopidogrel-Hydrogensulfat.

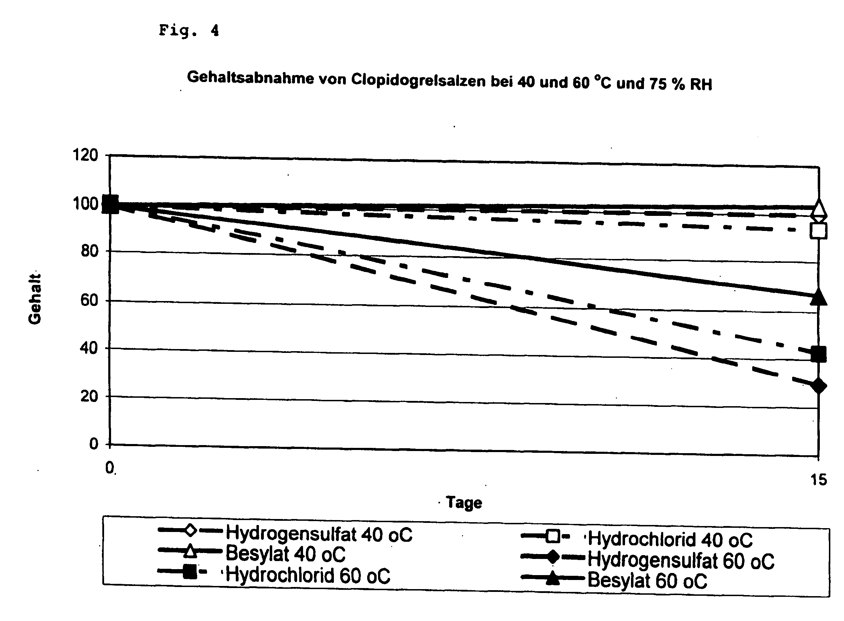

4.2 Darüber hinaus wurde die Gehaltsabnahme von Clopidogrel-Hydrogensulfat, - Hydrochlorid und -Besylat (kristallin) bei 40° und 60° C und 75% relativer Luftfeuchtigkeit über 15 Tage untersucht. Die Ergebnisse sind in der anliegenden Figur 4 wiedergegeben.

Man erkennt, dass sowohl bei 40° als auch bei 60° C das Besylatsalz (Clopidogrel-Benzolsulfonat) die beste Stabilität aufweist.

### Beispiel 5

### Adsorbat von (S)-(+)-Clopidogrel-Besylat an Calciumgluconat als Trägermaterial

Zu einer Lösung von 19,7 g (61,4 mmol) (S)-(+)-Clopidogrel in 300 ml wasserfreiem Diethylether wird bei 3° C langsam (ca. 30 min.) unter starkem Rühren eine Lösung von 11 g (69,5 mmol) wasserfreier Benzolsulfonsäure in 100 ml kalten, wasserfreien Diethylether getropft. Anschließend wird eine vorbereitete Aufschlämmung von 28 g Calciumgluconat in kaltem, wasserfreien Diethylether langsam zugegeben. Der nach Beendigung der Zugabe entstandene Kristallbrei wird abgenutscht, mit eiskaltem, wasserfreien Diethylether gewaschen und anschließend getrocknet.

Man erhält ein weißes, rieselfähiges Pulver.

### Beispiel 6

### Adsorbat von (S)-(+)-Clopidogrel-Besylat an Kieselgel/Mannit als Trägermaterial

Bei einer Temperatur von 2° - 3° C werden 20 g (62,3 mmol) (S)-(+)-Clopidogrel und 11 g (69,5 mmol) wasserfreie Benzolsulfonsäure in 200 ml wasserfreiem Diethylether zur Reaktion gebracht. Anschließend wird eine Aufschlämmung von 2 g Kieselsäure und 20 g Mannit in 100 ml wasserfreiem Diethylether langsam zugegeben. Das entstandene Adsorbat wird in der Kälte abgenutscht, mit eiskaltem, wasserfreien Diethylether gewaschen und anschließend getrocknet.

Man erhält 39 g eines weißen, rieselfähigen Pulvers.

### Beispiel 7 (nicht erfindungsgemäß)

### Adsorbat von (S)-(+)-Clopidogrel-Mesylat an Kieseigel/Mannit als Trägermaterial

Zu einer Lösung von 19,5 g (60,7 mmol) (S)-(+)-Clopidogrel in 300 ml wasserfreiem Diethylether wird bei 3°C langsam (ca. 30 min.) eine Lösung von 5,85 g (60,8 mmol) wasserfreier Methansulfonsäure in 100 ml kaltem, wasserfreiem Diethylether getropft. Anschließend wird eine vorbereitete Aufschlämmung von 1,95 g Kieselsäure und 19,5 g Mannit in kaltem, wasserfreiem Diethylether langsam zugegeben. Das nach Beendigung der Zugabe entstandene Adsorbat wird abgenutscht, mit eiskaltem, wasserfreien Diethylether gewaschen und anschließend getrocknet.

Man erhält 30 g eines rieselfähigen, weißen Pulvers.

### Belspiel 8 (nicht erfindungsgemäß)

### Adsorbat von (S)-(+)-Clopidogrel-Mesylat an Mannit als Trägermaterial

19,5 g (60,7 mmol) (S)-(+)-Clopidogrel und 5,85 g (60,8 mmol) Methansulfonsäure werden analog zum Beispiel 7 zur Reaktion gebracht. Anschließend wird eine vorbereitete Aufschlämmung von 19,5 g Mannit in kaltem, wasserfreien Diethylether langsam zugegeben. Das nach Beendigung der Zugabe entstandene Adsorbat wird abgenutscht, mit eiskaltem, wasserfreien Diethylether gewaschen und anschließend getrocknet.
Man erhält 29,7 g eines rieselfähigen, weißen Pulvers.

### Beispiel 9 (nicht erfindungsgemäß)

### Adsorbat von (S)-(+)-Clopidogrel an Kieselgel/Maisstärke als Trägermaterial

Zu einer Suspension von 2 g Aerosil 200 in CH₂Cl₂ wird eine Lösung von 5 g (15,6 mmol) (S)-(+)-Clopidogrel in wasserfreiem Dichlormethan langsam zugetropft. Nach 1 h wird eine Suspension von 4 g gelatinisierter Maisstärke in wasserfreiem Dichlormethan unter Rühren zugegeben. Nach Beendigung der Zugabe wird das Lösungsmittel abgezogen, wobei ein rein weißer Feststoff erhalten wird, der anschließend 12 h im Vakuum getrocknet wird.

Man erhält ein rein weißes, rieselfähiges Pulver mit einer Wirkstoffbeladung von 45,5%.

Die Wiederholung des Versuchs unter Verwendung von 8 g gelatinisierter Maisstärke ergibt ein rieselfähiges Pulver mit einer Wirkstoffbeladung von 33,3%.

### Beispiel 10

Es wurden zwei verschiedene Verfahren zur Herstellung von Adsorbaten der Clopidogrel-Salze angewandt. Im ersten Verfahren wird das Salz in einem geeigneten Lösungsmittel gelöst und in dieser Lösung wird das Adsorbtionsmittel suspendiert.

In einer zweiten Versuchsreihe wurde die Clopidogrel-Base in einem geeigneten Lösungsmittel gelöst, das Adsorbtionsmittel wurde zugegeben und das Salz auf das Trägermaterial ausgefällt.

In allen Versuchen wurden jeweils Lactose (Lactopress®), Mannit (Mannogem®) und Cellulose (Celphere®) als Adsorbtionsmittel eingesetzt.

Folgende Versuche wurden durchgeführt:

### Clopldogrel-Salz-Adsorbate mit Isolierung des Salzes

### a) Clopidogrel-Besylat-Adsorbate

Es werden 1,5 g (3,1 mmol) Clopidogrel-Besylat in 20 ml Aceton gelöst und 1,5 g Adsorbtionsmittel zugegeben. Das Lösungsmittel wird abgezogen, der Rückstand kurz mit MTB-Ether aufgeschlämmt und anschließend im Vakuum getrocknet.

### b) Clopidogrel-Hydrochlorid-Adsorbate

500 mg (1,4 mmol) Clopidogrel-Hydrochlorid werden in 10 ml Aceton gelöst. 500 mg Adsorbtionsmittel werden zugegeben und verrührt. Das Lösungsmittel wird abgezogen. Der Rückstand wird im Vakuum getrocknet.

### c) Clopidogrel-Hydrogensulfat-Adsorbate

500 mg (1,2 mmol) Clopidogrel-Hydrogensulfat werden in 10 ml Aceton gelöst. 500 mg Adsorbtionsmittel werden zugegeben und verrührt. Das Lösungsmittel wird abgezogen. Der Rückstand wird im Vakuum getrocknet.

### Clopidogrel-Salz-Adsorbate ohne vorherige Isolierung der Salze

### 1. Lösungsmittel Diethylether

### a) Clopidogrel-Besylat-Adsorbate

4,018 g (12,5 mmol) Clopidogrel-Base werden in 20 ml Diethylether gelöst. 6 g Adsorbtionsmittel und 1,977 g (12,5 mmol) Benzolsulfonsäure werden in 20 ml Ether zugegeben. Das Festprodukt wird abgesaugt, mit Ether gewaschen und im Vakuum getrocknet.

### b) Clopidogrel-Mesylat-Adsorbate

4,018 g (12,5 mmol) Clopidogrel-Base werden in 20 ml Diethylether gelöst. 6 g Adsorbtionsmittel und 1,2 g (12,5 mmol) Methansulfonsäure werden in 20 ml Ether zugegeben. Das Festprodukt wird abgesaugt, mit Ether gewaschen und im Vakuum getrocknet.

### c) Clopidogrel-Hydrochlorid-Adsorbate

3 g (9,3 mmol) Clopidogrel-Base werden in 31 ml Diethylether gelöst. 3 g Adsorbtionsmittel werden zugegeben und Chlorwasserstoffgas wird eingeleitet. Das Festprodukt wird abgesaugt, mit Ether gewaschen und im Vakuum getrocknet.

### 2. Lösungsmittel Methyl-tert-butylether (MTB-Ether)

### a) Clopidogrel-Besylat-Adsorbate

4,018 g (12,5 mmol) Clopidogrel-Base werden in 40 ml MTB-Ether gelöst. 6 g Adsorbtionsmittel und 1,977 g (12,5 mmol) Benzolsulfonsäure werden in 50 ml MTB-Ether zugegeben. Das Festprodukt wird abgesaugt, mit MTB-Ether gewaschen und im Vakuum getrocknet.

### b) Clopidogrel-Mesylat-Adsorbate

4,018 g (12,5 mmol) Clopidogrel-Base werden in 40 ml MTB-Ether gelöst. 6 g Adsorbtionsmittel und 1,2 g (12,5 mmol) Methansulfonsäure werden in 50 ml MTB-Ether zugegeben. Das Festprodukt wird abgesaugt, mit MTB-Ether gewaschen und im Vakuum getrocknet.

### Beispiel 11

Die Stabilität der gemäß Beispiel 10 erhaltenen Adsorbate wurde untersucht. Die Adsorbate bleiben bei Raumtemperatur pulverig und verändern ihre Farbe über mehr als zwei Monate nicht.

Die Abnahme des Wirkstoffgehalts bei einer Lagerung über 15 Tage bei 40° bzw. 60° C und 75% relativer Luftfeuchtigkeit wurde gemessen. Die Ergebnisse sind in der nachfolgenden Tabelle 5 [Gehalt nach 15 Tagen (Anfangswert normiert auf 100%)] zusammengefasst.

**Tabelle 5**

| **Clopidogrel-Hydrochlorid** | **40°C** | **60°C** |
|---|---|---|
| Reines Salz | 93,66 | 42,54 |
| Lactopress | 99,78 | 54,89 |
| Celpher | 92,81 | 43,74 |

| **Clopldogrel-Mesylat** | **40° C** | **60°C** |
|---|---|---|
| Reines Salz | 97,56 | 17,11 |
| Lactopress | 83,33 | 59,39 |
| Mannogem | 105,51 | 21,76 |

| **Clopidogrel-Besylat** | **40°C** | **60°C** |
|---|---|---|
| Reines Salz | 103,32 | 66,48 |
| Lactopress/Diethylether | 106,91 | 94,47 |
| Lactopress/MTB-Ether | 94,74 | 92,58 |

Man erkennt, dass die Adsorbate gegenüber den freien Salzen insbesondere bei erhöhter Temperatur eine größere Stabilität aufweisen.

### Beispiel 12

Gemäß Beispiel 10 hergestellte Adsorbate können direkt zu Tabletten verpresst werden. Dies wird durch die nachfolgenden Beispielformulierungen verdeutlicht. Die verwendeten Mengen der in den nachfolgenden Beispielen angegebenen weiteren Hilfsstoffe sind dem Fachmann aufgrund seines Basiswissens bekannt und können Standardwerken zur Formulierung von Tabletten, wie z. B. Ritschel et al., "Die Tablette", Editio Cantor - Aulendorf, 2. Aufl., 2002, entnommen werden.

### a) Clopidogrel-Besylat-Mikrokristalline Cellulose-Adsorbat

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Besylat -Mikrokristalline Cellulose-Adsorbat (entspricht 75 mg Clopidogrel Base) | 219,54 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 101 N |
| ■ | Abrieb: | 0,11 % |
| ■ | Zerfallzeit: | 65 Sek. |
| ■ | Freisetzung: | 100% nach 30 Min. |

Die so erhaltenen Tabletten können auch mit einem Überzug wie z. B. einem enterischen Überzug oder einem Überzug zur Geschmacksmaskierung versehen werden.

### b) Clopidogre/-Besylat-Mannit Adsorbat

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Besylat-Mannit-Adsorbat (entspricht 75 mg Clopidogrel Base) | 219,54 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 106 N |
| ■ | Abrieb: | 0,15% |
| ■ | Zerfallzeit: | 62 Sek. |
| ■ | Freisetzung: | 100% nach 30 Min. |

Die so erhaltenen Tabletten können mit einem Überzug wie z. B. einem enterischen Überzug oder einem Überzug zur Geschmacksmaskierung versehen werden.

### c) Clopidogrel-Besylat-Lactose-Adsorbat

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Besylat-Lactose-Adsorbat (entspricht 75 mg Clopidogrel Base) | 219,54 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 96 N |
| ■ | Abrieb: | 0,21% |
| ■ | Zerfallzeit: | 76 Sek. |
| ■ | Freisetzung: | 100% nach 30 Min. |

Die so erhaltenen Tabletten können mit einem Überzug wie z. B. einem enterischen Überzug oder einem Überzug zur Geschmacksmaskierung versehen werden.

### d) Clopidogrel-Mesylat-Mannitol-Adsorbat (nicht erfindungsgemäß)

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Mesylat-Mannitol-Adsorbat (entspricht 75 mg Clopidogrel Base) | 194,79 mg |
| ■ | Hilfsstoffe (Gleitmittel, Füllstoffe, Zerfallsbeschteuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 98 N |
| ■ | Abrieb: | 0,21% |
| ■ | Zerfallzeit: | 55 Sek. |
| ■ | Freisetzung: | 100% nach 30 Min. |

Die so erhaltenen Tabletten können mit einem Überzug wie z. B. einem enterischen Überzug oder einem Überzug zur Geschmacksmaskierung versehen werden.

### e) Clopidogrel-Mesylat-Lactose-Adsorbat (nicht erfindungsgemäß)

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Mesylat-Lactose-Adsorbat | 194,79 mg |
| ■ | Hilfsstoffe (Gleitmittel, Füllstoffe, Zerfallsbeschleuniger, Fließregulator, Netzmittel) | ad275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 88 N |
| ■ | Abrieb: | 0,22% |
| ■ | Zerfallzeit: | 72 Sek. |
| ■ | Freisetzung: | 100% nach 30 Min. |

Die so erhaltenen Tabletten können mit einem Überzug wie z. B. einem enterischen Überzug oder einem Überzug zur Geschmacksmaskierung versehen werden.

### f) Clopidogrel-HCl-Lactose-Adsorbat (nicht erfindungsgemäß)

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-HCl-Lactose-Adsorbat | 167,0 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 95 N |
| ■ | Abrieb: | 0,20% |
| ■ | Zerfallzeit: | 75 Sek. |
| ■ | Freisetzung: | 100% nach 30 Min. |

Die so erhaltenen Tabletten können mit einem Überzug wie z. B. einem enterischen Überzug oder einem Überzug zur Geschmacksmaskierung versehen werden.

### g): Clopidogrel-HCl-Mikrokristailline Cellulose-Adsorbat (nicht erfindungsgemäß)

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-HCl -Mikrokristalline Cellulose-Adsorbat (entspricht 75 mg Clopidogrel Base) | 167,0 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 100 N |
| ■ | Abrieb: | 0,13% |
| ■ | Zerfallzeit: | 65 Sek. |
| ■ | Freisetzung: | 100% nach 30 Min. |

Die so erhaltenen Tabletten können auch mit einem Überzug wie z. B. einem enterischen Überzug oder einem Überzug zur Geschmacksmaskierung versehen werden.

### h) Clopidogrel-Hydrogensulfat-Mikrokristailine Cellulose-Adsorbat (nicht erfindungsgemäß)

Aus dem Adsorbat wurden Clopidogrel-Tabletten mit einer Gesamtmasse von 275 mg mittels Direktverpressung nach folgender Zusammensetzung hergestellt:

| | | |
|---|---|---|
| ■ | Clopidogrel-Hydrogensulfat -Mikrokristalline Cellulose-Adsorbat (entspricht 75 mg Clopidogrel Base) | 195,75 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 108 N |
| ■ | Abrieb: | 0,12% |
| ■ | Zerfallzeit: | 78 Sek. |
| ■ | Freisetzung: | 98% nach 30 Min. |

Die so erhaltenen Tabletten können auch mit einem Überzug wie z. B. einem enterischen Überzug oder einem Überzug zur Geschmacksmaskierung versehen werden.

### i) Clopidogrel-Hydrogensulfat-Mannitol-Adsorbat (nicht erfindungsgemäß)

Aus dem Adsorbat wurden Clopidogrel- Tabletten mit einer Gesamtmasse von 275 mg mittels Direktverpressung nach folgender Zusammensetzung hergestellt:

| | | |
|---|---|---|
| ■ | Clopidogrel-Hydrogensulfat -Mannitol-Adsorbat (entspricht 75 mg Clopidogrel Base) | 195,75 mg |
| ■ | Hilfsstoffe (Gleitmittel, Füllstoffe, Zerfallsbeschleuniger, Fließregulator, Netzmittel) | ad 275 mg |

Eigenschaften der pressfertigen Mischung und der Tabletten:

| | | |
|---|---|---|
| ■ | Kompressibilität und Fließfähigkeit: | befriedigend bis gut |
| ■ | Mittlere Härte: | 110 N |
| ■ | Abrieb: | 0,13% |
| ■ | Zerfallzeit: | 80 Sek. |
| ■ | Freisetzung: | 98% nach 30 Min. |

Die so erhaltenen Tabletten können auch mit einem Überzug wie z. B. einem enterischen Überzug oder einem Überzug zur Geschmacksmaskierung versehen werden.

## Patentansprüche

1. Kristallines Clopidogrel-Besylat x ½ Dioxan.

2. Verfahren zur Herstellung von kristallinem Clopidogrel-Besylat x ½ Dioxan nach Anspruch 1, wobei das Salz aus einer Lösung des Clopidogrels ausgefällt wird und das Lösungsmittel Dioxan ist.

3. Verfahren zur Reinigung von Clopidogrel oder einem salz davon wobei verunreinigtes Clopidogrel oder ein Salz davon beim Auskristallisieren aus Dioxan in Clopidogrel-Besylat überführt wird.

4. Verfahren nach Anspruch 3, wobei aus dem verunreinigten Clopidogrel-Salz zunächst die Clopidogrel-Base freigesetzt wird.

5. Verfahren nach einem der Ansprüche 3 oder 4, wobei aus dem isolierten Clopidogrel-Besylat die Clopidogrel-Base freigesetzt wird oder das isolierte Clopidogrel-Besylat in ein anderes Salz überführt wird.

6. Verwendung des salzes nach Anspruch 1 zur Herstellung einer pharmazeutischen Formulierung.

## Claims

1. A crystalline clopidogrel besylate x ½ dioxane.

2. A method for preparing the crystalline clopidogrel besylate x ½ dioxane according to claim 1, wherein the salt is precipitated from a solution of clopidogrel and the solvent is dioxane.

3. A method for purifying clopidogrel or a salt thereof, wherein impure clopidogrel or a salt thereof is converted into clopidogrel besylate when crystallised from dioxane.

4. The method according to claim 3, wherein the clopidogrel base is first released from the impure clopidogrel salt.

5. The method according to either claim 3 or claim 4, wherein the clopidogrel base is released from the isolated clopidogrel besylate or the isolated clopidogrel besylate is converted into another salt.

6. A use of the salt according to claim 1 for preparing a pharmaceutical formulation.

## Revendications

1. Bésylate de clopidogrel x ½ dioxane, sous forme cristalline.

2. Procédé de préparation de bésylate de clopidogrel x ½ dioxane, sous forme cristalline, selon la revendication 1, le sel étant précipité à partir d'une solution dudit clopidogrel et le solvant étant du dioxane.

3. Procédé de purification de clopidogrel ou de l'un de ses sels, dans lequel du clopidogrel contaminé ou l'un de ses sels est transformé, lors de sa cristallisation à partir d'une solution dans du dioxane, en bésylate de clopidogrel.

4. Procédé selon la revendication 3, dans lequel ledit clopidogrel contaminé est préalablement transformé en base libre de clopidogrel.

5. Procédé l'une des revendication 3 ou 4, dans lequel le bésylate de clopidogrel est transformé, suite à son isolation, en base libre de clopidogrel ou le bésylate de clopidogrel est transformé, suite à son isolation, en un autre sel.

6. Utilisation du sel selon la revendication 1 pour préparer une formulation pharmaceutique.
